# FASCICULE DE BREVET EUROPEEN

(11) **EP 3 363 518 B1**
(45) Date de publication et mention de la délivrance du brevet: **25.03.2020**
(21) Numéro de dépôt: 18153418.1
(22) Date de dépôt: 25.01.2018
(51) Int. Cl.: B01D 35/06

(54) **PROCÉDÉ ET SYSTÈME DE DÉTECTION DE DÉBRIS POUR TURBOMACHINE**
VERFAHREN UND SYSTEM ZUM NACHWEIS VON RÜCKSTÄNDEN FÜR TURBOMASCHINE
METHOD AND SYSTEM FOR DETECTING DEBRIS FOR A TURBINE ENGINE

(30) Priorité: 15.02.2017 BE 1705096
(43) Date de publication de la demande: 22.08.2018
(73) Titulaire: Safran Aero Boosters SA, 4041 Herstal (BE)
(72) Inventeur: RAIMARCKERS, Nicolas, 4263 Tourinne (Braives) (BE)
(74) Mandataire: Lecomte & Partners

(56) Documents cités:
- EP-A1- 3 127 592
- WO-A1-2015/134602
- GB-A- 2 004 374

## Description

### Domaine technique

L'invention se rapporte à la surveillance d'organes mécaniques lubrifiés dans une turbomachine. Plus précisément, l'invention concerne la détection de débris métalliques dans de l'huile d'une turbomachine. L'invention a également trait à une turbomachine axiale, notamment un turboréacteur d'avion ou un turbopropulseur d'aéronef. L'invention a également pour objet un procédé de détection de débris ferromagnétiques.

### Technique antérieure

La présence de débris métalliques dans l'huile d'un circuit de lubrification témoigne de l'usure des éléments mobiles d'une turbomachine. Ainsi, en analysant la quantité et la taille des débris métalliques en circulation dans l'huile, il est possible d'estimer la santé du moteur. En particulier, une augmentation brusque du nombre de débris détectés peut signifier qu'un roulement ou qu'un engrenage s'use prématurément. Dès lors, une maintenance doit être planifiée afin d'éviter une panne ou une casse mécanique.

Le document US4731578A divulgue un système de détection électrique de présence de particules ferreuses dans un fluide. Ce système comporte à la fois un aimant et une bobine électrique. En fonctionnement, les particules ferreuses sont captées par l'aimant et s'y accumulent. Toutefois, la précision d'analyse de ce système reste limitée.

Le document US5528138A divulgue un système de détection de débris ferromagnétiques dans un flux. Un module magnétique comprend une bobine accolée à une crépine. Un autre système de détection est divulgué dans le document EP-3127592.

### Résumé de l'invention

### Problème technique

L'invention a pour objectif de résoudre au moins un des problèmes posés par l'art antérieur. Plus précisément, l'invention a pour objectif d'améliorer la précision de contrôle d'un flux de lubrifiant. L'invention a également pour objectif de proposer une solution simple, résistante, légère, économique, fiable, facile à produire, commode d'entretien, d'inspection aisée, et améliorant le rendement.

### Solution technique

L'invention a pour objet un système de détection de débris ferromagnétiques dans un flux, notamment un flux d'huile dans un circuit de lubrification de turbomachine, le système comprenant : un passage destiné à être traversé par le flux ; un module de détection magnétique de débris ferromagnétiques du flux, le module comportant : une moitié amont, une moitié aval, un premier aimant, une bobine associée au premier aimant et disposée dans la moitié amont; remarquable en ce qu'il comprend en outre une première crépine disposée dans le passage du côté de la moitié amont du module de détection afin d'y filtrer des débris ferromagnétiques. Les moitiés sont considérées longitudinalement. Le premier aimant reste optionnel.

Selon des modes avantageux de l'invention, le système peut comprendre une ou plusieurs des caractéristiques suivantes, prises isolément ou selon toutes les combinaisons techniques possibles :
Le système comprend en outre un deuxième aimant disposé entre la première crépine et le module de détection.

Le module de détection comprend un élément formant un pôle magnétique, ledit élément étant en contact du premier aimant et la bobine étant enroulée autour dudit élément.

L'élément magnétique comprend une face en contact du premier aimant et une face en regard de la première crépine, et éventuellement du deuxième aimant.

Le système comprend en outre une deuxième crépine disposée dans le passage afin d'y intercepter des débris, le module de détection étant disposé entre la première crépine et la deuxième crépine.

La première crépine comprend des premières mailles, et la deuxième crépine comprend des deuxièmes mailles plus fines que les premières mailles.

Le système comprend en outre un troisième aimant disposé en aval du module de détection.

Le passage est configuré de manière à former un vortex dans le flux de manière à séparer les débris ferromagnétiques en fonction de leurs masses.

Le système comprend une unité de traitement d'un signal électrique de la bobine, ladite unité de traitement étant adaptée pour reconnaître le mouvement d'une particule ferromagnétique lorsque le signal électrique de la bobine dépasse un seuil prédéterminé.

La bobine forme longitudinalement un tronçon électromagnétique, éventuellement en combinaison d'une carcasse électromagnétique, ledit tronçon formant une extrémité longitudinale du module.

Le passage forme un segment étanche, au moins de la première crépine au module.

Le premier aimant s'étend majoritairement depuis l'élément vers l'opposé de la première crépine.

Le premier aimant est plus fort et/ou plus lourd que le deuxième aimant et/ou que le troisième aimant.

Le troisième aimant est plus fort et/ou plus lourd que le deuxième aimant.

Le troisième aimant est disposé entre le module de détection et la deuxième crépine.

La bobine est configurée de sorte à mesurer une variation de champ magnétique dans le flux lors du passage d'une particule ferromagnétique.

Le module est disposé dans le passage de manière à y détecter des débris ferromagnétiques.

Le module forme une réduction de section passante dans le passage, éventuellement de manière à y accélérer le flux.

Le passage est droit, notamment de la première crépine au module, et éventuellement jusqu'à la deuxième crépine.

La première et/ou la deuxième crépine est adaptée pour séparer des débris ferromagnétiques du flux traversant le passage.

Le flux présente une vitesse et/ou un débit connu, par exemple en fonction d'une pompe qui l'entraîne dans le passage.

La ou chaque bobine est séparée de la ou de chaque crépine,

Un espace sépare axialement, notamment selon l'écoulement du flux, chaque bobine de chaque crépine.

L'invention a également pour objet un circuit de lubrification comprenant un sens d'écoulement, notamment dans un passage ; et un module de détection de débris ferromagnétiques qui comporte : un premier aimant, une bobine associée au premier aimant, remarquable en ce qu'il comprend en outre une première crépine disposée avant le module, et/ou une deuxième crépine après le module selon le sens d'écoulement.

L'invention a également pour objet une turbomachine, notamment un turboréacteur, comprenant un circuit de lubrification avec un système de détection de débris ferromagnétiques, remarquable en ce que le système est conforme à l'invention, préférentiellement le circuit de lubrification comporte au moins une pompe, un réservoir, et un sens d'écoulement, la première crépine étant disposée à distance et en amont du module de détection.

Selon des modes avantageux de l'invention, la turbomachine peut comprendre une ou plusieurs des caractéristiques suivantes, prises isolément ou selon toutes les combinaisons techniques possibles :
La pompe comprend un corps, le passage étant formé dans ledit corps, éventuellement chaque module de détection de débris ferromagnétiques est à distance du réservoir.

La première crépine et/ou le deuxième aimant comprennent des moyens de fixation réversibles configurés pour le/les monter dans le passage.

Le circuit de lubrification comprend plusieurs pompes de récupération, la turbomachine comprenant plusieurs systèmes de détection conformes à l'invention et disposés en amont de chaque pompe de récupération.

La turbomachine comprend un réseau électrique branchant électriquement les bobines les unes aux autres.

La turbomachine comprend une enceinte de lubrification dans laquelle est disposé un palier lubrifié par le circuit.

L'invention a également pour objet un procédé de détection de débris ferromagnétiques dans un flux traversant un passage, notamment de l'huile de lubrification d'une turbomachine, le procédé comprenant les étapes suivantes : (a) circulation du flux au travers du passage ; et (b) détection de débris ferromagnétiques dans le flux à l'aide d'un système de détection de débris ferromagnétiques, remarquable en ce que le système est conforme à l'invention; les débris ferromagnétiques comprennent éventuellement, par ordre décroissant de tailles, des premiers débris, des deuxièmes débris, des troisièmes débris et des quatrièmes débris.

Selon des modes avantageux de l'invention, le procédé de détection peut comprendre une ou plusieurs des caractéristiques suivantes, prises isolément ou selon toutes les combinaisons techniques possibles :
La première crépine est configurée pour bloquer les premiers débris et pour laisser passer les deuxièmes débris.

Le deuxième aimant est configuré pour bloquer les quatrièmes débris, et éventuellement pour laisser passer les troisièmes débris et les deuxièmes débris.

Le premier aimant est configuré pour bloquer les troisièmes débris, et éventuellement pour laisser passer les deuxièmes débris.

La bobine du module de détection est configurée pour détecter le mouvement des deuxièmes débris et/ou la présence des troisièmes débris bloqués par le module de détection, éventuellement les deuxièmes débris ont une taille moyenne de 750 µm.

Le troisième aimant et/ou la deuxième crépine sont configurés pour bloquer les deuxièmes débris.

Le système comprend des moyens de blocage des deuxièmes débris.

Lorsqu'un aimant bloque ou laisse passer un type de débris, il peut s'agir de la majorité ou d'au moins 80% de ces débris.

De manière générale, les modes avantageux de chaque objet de l'invention sont également applicables aux autres objets de l'invention. Chaque objet de l'invention est combinable aux autres objets, et les objets de l'invention sont également combinables aux modes de réalisation de la description, qui en plus sont combinables entre eux, selon toutes les combinaisons techniques possibles.

### Avantages apportés

Combiner des crépines et des aimants avec un module de détection permet de modifier la population des débris présents dans le flux. Les écarts types des tailles et des masses des débris peuvent se réduire ce qui simplifie le traitement des données fournies. L'analyse des probabilités de pannes est simplifiée. Le système devient plus sûr.

En outre, lorsque les débris s'agglomèrent, ils forment des couches moins denses, ce qui réduit le risque de colmatage des crépines et la perte de charge qui s'en suit. Le calibrage évolutif nécessaire au niveau d'un même module de détection est facilité.

### Brève description des dessins

La figure 1 représente une turbomachine axiale selon l'invention.
La figure 2 est un schéma d'un système de détection selon l'invention.
La figure 3 illustre un diagramme du procédé de détection de débris selon l'invention.

### Description des modes de réalisation

Dans la description qui va suivre, le terme « aimant » renvoie à un aimant permanent. La direction longitudinale est considérée selon le sens d'écoulement du flux au travers du passage. L'amont et l'aval sont entendus en relation avec le sens d'écoulement du flux.

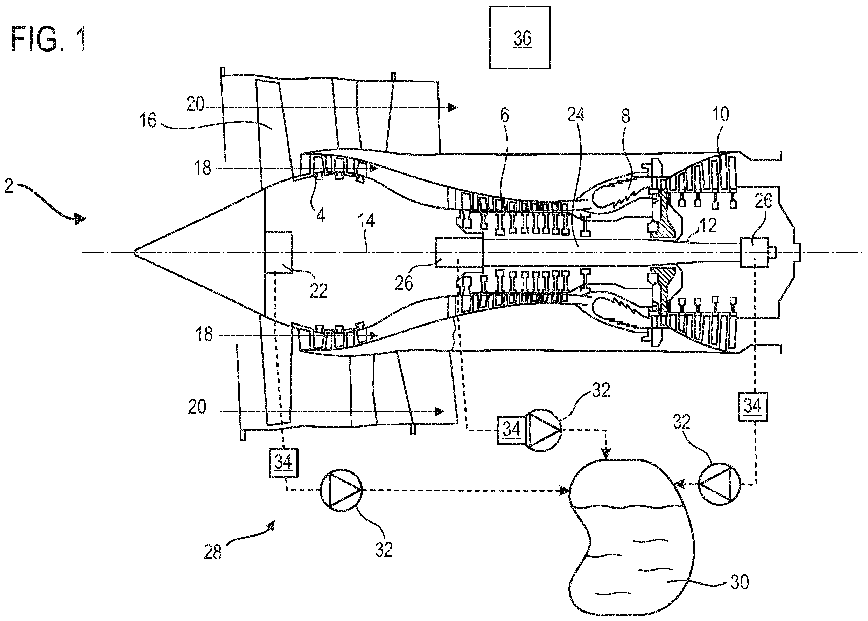

La figure 1 représente de manière simplifiée une turbomachine axiale. Il s'agit dans ce cas précis d'un turboréacteur double-flux. Le turboréacteur 2 comprend un premier niveau de compression, dit compresseur basse pression 4, un deuxième niveau de compression, dit compresseur haute pression 6, une chambre de combustion 8 et un ou plusieurs niveaux de turbines 10. En fonctionnement, la puissance mécanique des turbines 10 transmise via des arbres jusqu'au rotor 12 met en mouvement les deux compresseurs 4 et 6. Ces derniers comportent plusieurs rangées d'aubes de rotor associées à des rangées d'aubes de stators. La rotation du rotor autour de son axe de rotation 14 permet ainsi de générer un débit d'air et de comprimer progressivement ce dernier jusqu'à l'entrée dans la chambre de combustion 8.

Un ventilateur d'arrivée communément désigné fan ou soufflante 16 est couplé au rotor 12 et génère un flux d'air qui se divise en un flux primaire 18 traversant les différents niveaux susmentionnés de la turbomachine, et un flux secondaire 20 traversant un conduit annulaire. Des moyens de démultiplication, tel un réducteur épicycloïdal 22, peuvent réduire la vitesse de rotation de la soufflante 16 et/ou du compresseur basse pression 4 par rapport au niveau de turbine 10 associé. Le flux secondaire 20 peut être accéléré de sorte à générer une réaction de poussée utile au vol d'un avion.

Le rotor 12 comporte plusieurs arbres 24 concentriques qui sont articulés à l'aide de paliers 26. Le refroidissement et/ou la lubrification des paliers 26 et de l'optionnel réducteur épicycloïdal 22 sont assurés par un circuit de lubrification 28, éventuellement fermé. Ce circuit de lubrification 28 peut être propre au turboréacteur 2. Le circuit de lubrification 28 peut en outre alimenter des actionneurs tels des vérins (non représentés). Le circuit de lubrification 28 peut également comprendre un échangeur de chaleur pour refroidir l'huile qui peut dépasser les 200°C, et même 230°C dans certains cas. Ces températures amplifient l'agressivité de l'huile corrosive vis-à-vis des joints et des parties polymères en général.

Le circuit de lubrification 28 peut comprendre des conduites de récupération d'huile collectant l'huile dans les enceintes de lubrification des paliers 26 et l'acheminant dans le réservoir 30. Il peut également comporter une conduite de récupération de l'huile lubrifiant le réducteur épicycloïdal 22 et retournant cette huile dans le réservoir 30.

Afin de forcer la circulation de l'huile lors de sa récupération, le circuit de lubrification 28 peut comprendre plusieurs pompes 32 permettant d'aspirer l'huile depuis leur chambre de lubrification, puis de la refouler dans le réservoir 30. En complément, la turbomachine 2 comprend au moins un système de détection 34, préférentiellement plusieurs systèmes de détection 34 qui sont chacun associés à une pompe 32. Chaque système de détection 34 permet de détecter la présence et/ou la circulation de débris ferromagnétiques, ou particules ferromagnétiques, contenus dans l'huile. Ces débris peuvent notamment résulter d'une usure d'un palier ou d'une dent d'engrenage formant le réducteur 22.

Une unité de traitement 36 de signal électrique peut être branchée à au moins un système de détection 34, ou à chaque système de détection 34. Dès lors, l'unité 36 parvient à identifier la présence de débris au niveau de chaque conduite, et donc de la zone de collecte correspondante, par exemple lorsque chaque module code son signal électrique. Les débris peuvent avoir une taille comprise entre 50µm et 1 000µm, ou entre 150µm et 750µm.

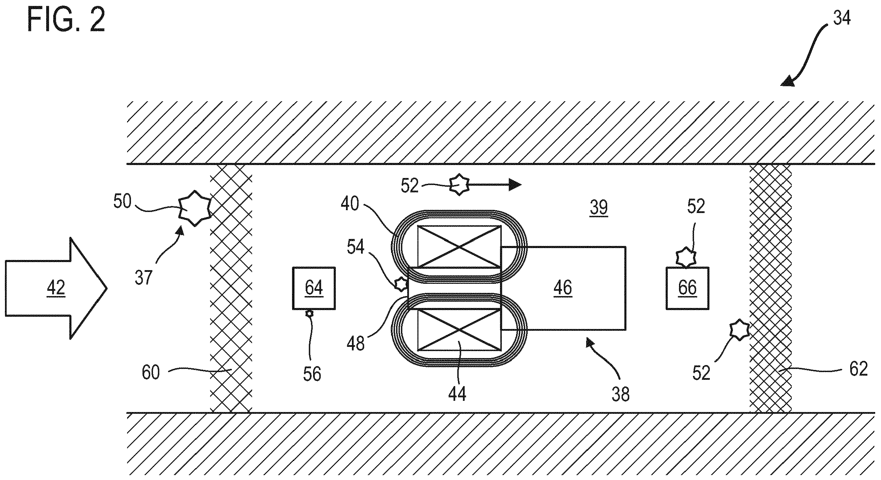

La figure 2 montre un système de détection 34. Il peut correspondre à ceux de la figure 1.

Le système de détection 34 comprend avantageusement un module de détection magnétique 38 de débris ferromagnétiques 37. Il occupe un passage 39 traversé par le flux 42. Le passage peut être matérialisé par une conduite ou par une paroi d'un corps. Ce module 38 est apte à mesurer le champ magnétique 40 dans le flux 42 d'huile. Une variation de champ magnétique 40 est décelée lorsqu'une particule ferromagnétique est présente et/ou se déplace dans le flux. Le flux peut être mesuré par une bobine électromagnétique 44. Le champ magnétique 40 peut être produit par un premier aimant 46. En remplacement du premier aimant, le champ magnétique 40 peut être généré par une deuxième bobine électromagnétique.

En complément, le module de détection 38 peut comprendre un élément magnétique 48 formant un pôle magnétique. Cet élément magnétique 48 peut être à l'intérieur de la bobine électromagnétique 44, et la traverser. Il peut toucher le premier aimant 46, et s'étendre en amont de la bobine 44. Le module de détection 38 montre une moitié amont dans laquelle est placée la bobine électromagnétique 44. Cette dernière peut former la face amont du module de détection 38 à l'aide de sa carcasse ferromagnétique. Elle peut également toucher le premier aimant 46 grâce à sa face aval.

Lors du fonctionnement de la turbomachine, le flux 42 transporte des débris ferromagnétiques 37 de plusieurs tailles. Les débris ferromagnétiques 37 peuvent comprendre des premiers débris 50 plus grands que des deuxièmes débris 52. En outre, les débris ferromagnétiques 37 peuvent comprendre, par ordre décroissant de tailles, des troisièmes débris 54, éventuellement des quatrièmes débris 56, et optionnellement des cinquièmes débris. Ces troisièmes débris 54, et quatrièmes débris 56 étant plus petits que les deuxièmes débris 52. Les caractéristiques de tailles des débris ferromagnétiques (50-56) peuvent être remplacées par les masses des débris ferromagnétiques (50-56). Ainsi, l'ensemble des débris (50-56) transportés par le flux 42 peut être divisé en sous-ensembles définis par des masses moyennes ou des tailles moyennes de particule. Les sous-ensembles peuvent avoir des longueurs de plages égales, et/ou comprendre des bornes de plages communes.

Le système de détection 34 comprend une première crépine 60 en amont, et éventuellement une deuxième crépine 62 en aval. Chaque crépine (60 ; 62) peut former un filtre. Elles séparent les débris (50-56) du flux 42, et les retiennent. Au moins une d'elle peut être un treillis, par exemple plan, tubulaire ; ou un bloc de mousse à pores ouvertes. Le module de détection 38 est disposé entre et à distance des crépines (60 ; 62). Chaque crépine (60 ; 62) peut traverser le passage 39, et/ou y former une barrière à débris 37. Par ailleurs, Les crépines (60 ; 62) peuvent présenter des mailles passantes de tailles différentes. La première crépine 60 peut présenter des mailles plus grandes que celles de la deuxième crépine 62 pour bloquer les premiers débris 50 tout en laissant passer les débris plus fins. La taille des mailles de la première crépine 60 peut être supérieure ou égale à 1000µm, ou à 750µm. En revanche, les mailles de la deuxième crépine 62 bloquent les deuxièmes débris 52 et/ou les troisièmes débris 56.

Le système de détection 34 peut comprendre un deuxième aimant 64 en amont, et/ou un troisième aimant 66 en aval du module. Ces aimants (64 ; 66) sont intercalés entre le module de détection 38 et les crépines (60 ; 62). En fonctionnement, le premier aimant 64 intercepte les plus petits débris, par exemple les quatrièmes débris 56, et laisse passer les plus gros en raison de leurs énergies cinétiques communiquées par le flux 42. De son côté, le troisième aimant 66 peut bloquer les deuxièmes débris 52 ; ce qui peut épauler l'effet de la deuxième crépine 62. Eventuellement, le groupe des deuxièmes débris est subdivisé en deux populations donc les débris les plus fins sont arrêtés par le troisième aimant 66, et les débris les plus gros sont bloqués par la deuxième crépine 62.

En fonctionnement, les troisièmes débris 54 sont captés par le module de détection 38, notamment contre l'élément 48 en raison de son interaction magnétique avec le premier aimant 46. Leur présence influe statiquement sur le flux magnétique 40. En revanche, les deuxièmes débris 52 contournent le module de détection 38 et poursuivent leurs déplacements poussés par le flux 42. Au niveau de la bobine 44, elles modifient le flux 40 et créent des discontinuités dans le signal du module de détection 38, par exemple dans le signal électrique que la bobine 44 contribue à produire et/ou à mesurer. Lorsque le signal comprend un pic qui dépasse un seuil donné prédéfini, le système de détection 34 reconnaît qu'une particule ferromagnétique, en l'occurrence une deuxième particule 52, est passée. Le système de détection 34 peut être calibré de sorte que la taille des deuxièmes débris corresponde à une taille critique signifiant qu'un incident mécanique particulier est survenu.

Le système de détection 34 peut être configuré pour former un vortex dans le passage 39. Ce passage 39 peut comprendre une enceinte avec une entrée désaxée, et/ou des ailettes (non représentées) provoquant un mouvement de rotation du flux 42. Ainsi, le vortex participe à la séparation des débris, grâce à leurs masses et à leurs inerties. Ceci facilite à nouveau la lecture des caractéristiques des débris dans le flux puisqu'ils sont triés à l'approche de la bobine 44.

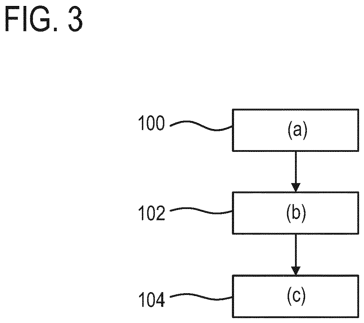

La figure 3 esquisse un diagramme du procédé de détection de débris ferromagnétiques 37 à l'aide d'un système de détection, par exemple dans une turbomachine. La turbomachine et le système de détection peuvent correspondre à ceux décrits en figures 1 et 2.

Le procédé peut comprendre les étapes suivantes ; éventuellement réalisées dans l'ordre qui suit :
(a) circulation 100 du flux au travers du passage ; et
(b) détection 102 de débris ferromagnétiques dans le flux à l'aide du système de détection de débris ferromagnétiques ; et
(c) identification 104 d'une anomalie mécanique et/ou déclenchement d'une alarme.

Lors de l'étape (a) circulation 100, la première crépine est configurée pour bloquer les premiers débris, et est traversée par les deuxièmes débris ainsi que les débris plus petits. Le deuxième aimant bloque les quatrièmes débris tandis qu'il laisse libres les débris avec une énergie cinétique plus importante ; par exemple les deuxièmes débris et les troisièmes débris. Ces derniers sont attirés magnétiquement contre le module de détection, par exemple au centre contre l'élément magnétique.

Le passage des deuxièmes débris modifie le champ magnétique dans le flux au voisinage de la bobine, ce qui conduit à effectuer l'étape (b) détection 102 des débris. Cette étape permet également d'estimer la taille et/ou la masse des débris ferromagnétiques.

En comparant les tailles des débris détectés par rapport à une taille critique, une situation atypique peut être identifiée. Leurs dangerosités peuvent être estimées. Lorsque la taille et/ou la masse d'une ou de plusieurs débris représente un danger significatif, une anomalie peut être reconnue à l'étape (c) identification 104. Une alarme peut être déclenchée.

## Revendications

1. Système de détection (34) de débris ferromagnétiques (37) dans un flux (42), notamment un flux d'huile dans un circuit de lubrification (28) d'une turbomachine (2), le système de détection (34) comprenant :
- un passage (39) destiné à être traversé par le flux (42);
- un module de détection magnétique (38) de débris ferromagnétiques (37) du flux (42), le module (38) comportant : une moitié amont, une moitié aval, un premier aimant (46), une bobine (44) associée au premier aimant (46) et disposée dans la moitié amont;
**caractérisé en ce qu'**il comprend en outre
une première crépine (60) disposée dans le passage (39) du côté de la moitié amont du module de détection (38) afin d'y filtrer des débris ferromagnétiques (37).

2. Système de détection (34) selon la revendication 1, **caractérisé en ce qu'**il comprend en outre un deuxième aimant (64) disposé entre la première crépine (60) et le module de détection (38).

3. Système de détection (34) selon l'une des revendications 1 à 2, **caractérisé en ce que** le module de détection (38) comprend un élément (48) formant un pôle magnétique, ledit élément (48) étant en contact du premier aimant (46), et la bobine (44) étant enroulée autour dudit élément (48).

4. Système de détection (34) selon la revendication 3, **caractérisé en ce que** l'élément (48) comprend une face en contact du premier aimant (46) et une face en regard de la première crépine (60), et éventuellement du deuxième aimant (64).

5. Système de détection (34) selon l'une des revendications 1 à 4, **caractérisé en ce qu'**il comprend en outre une deuxième crépine (62) disposée dans le passage (39) afin d'y intercepter des débris, le module de détection (38) étant disposé entre la première crépine (60) et la deuxième crépine (62).

6. Système de détection (34) selon la revendication 5, **caractérisé en ce que** la première crépine (60) comprend des premières mailles, et la deuxième crépine (62) comprend des deuxièmes mailles plus fines que les premières mailles.

7. Système de détection (34) selon l'une des revendications 1 à 6, **caractérisé en ce qu'**il comprend en outre un troisième aimant (66) disposé en aval du module de détection (38).

8. Système de détection (34) selon l'une des revendications 1 à 7, **caractérisé en ce que** le passage (39) est configuré de manière à former un vortex dans le flux (42) de manière à séparer les débris ferromagnétiques (37) en fonction de leurs masses.

9. Système de détection (34) selon l'une des revendications 1 à 8, **caractérisé en ce qu'**il comprend une unité de traitement (36) d'un signal électrique de la bobine (44), ladite unité de traitement (36) étant adaptée pour reconnaître le mouvement d'une particule ferromagnétique lorsque le signal électrique de la bobine (44) dépasse un seuil prédéterminé.

10. Turbomachine (2), notamment un turboréacteur, comprenant un circuit de lubrification (28) avec un système de détection (34) de débris ferromagnétiques (37), **caractérisée en ce que** le système (34) est conforme à l'une des revendications 1 à 9, préférentiellement le circuit de lubrification (28) comporte au moins une pompe (32), un réservoir (30), et un sens d'écoulement, la première crépine (60) étant disposée à distance et en amont du module de détection (38).

11. Turbomachine (2) selon la revendication 10, **caractérisée en ce que** la pompe (32) comprend un corps, le passage (39) étant formé dans ledit corps, éventuellement chaque module de détection (38) de débris ferromagnétiques (37) est à distance du réservoir (30).

12. Procédé de détection de débris ferromagnétiques (37) dans un flux (42) traversant un passage (39), notamment de l'huile de lubrification d'une turbomachine, le procédé comprenant les étapes suivantes : (a) circulation (100) du flux au travers du passage (39) ; et (b) détection (102) de débris ferromagnétiques dans le flux (42) à l'aide d'un système de détection (34) de débris ferromagnétiques (37), **caractérisé en ce que** le système est conforme à l'une des revendications 1 à 9 ; les débris ferromagnétiques comprennent éventuellement, par ordre décroissant de tailles, des premiers débris (50), des deuxièmes débris (52), des troisièmes débris (54) et des quatrièmes débris (56).

13. Procédé selon la revendication 12, **caractérisé en ce que** la première crépine (60) est configurée pour bloquer les premiers débris (50) et pour laisser passer les deuxièmes débris (52) ; et **en ce que** le premier aimant (46) est configuré pour bloquer les troisièmes débris (54), et éventuellement pour laisser passer les deuxièmes débris (52) ; et **en ce que** le système comprend des moyens de blocage des deuxièmes débris (52).

14. Procédé selon l'une des revendications 12 à 13, et système selon l'une des revendications 2 à 9, **caractérisé en ce que** le deuxième aimant (64) est configuré pour bloquer les quatrièmes débris (56), et éventuellement pour laisser passer les troisièmes débris (54) et les deuxièmes débris (52).

15. Procédé selon l'une des revendications 12 à 14, **caractérisé en ce que** la bobine (44) du module de détection (38) est configurée pour détecter le mouvement des deuxièmes débris (52) et/ou la présence de troisièmes débris (54) bloqués par le module de détection, éventuellement les deuxièmes débris (52) ont une taille moyenne de 750 µm.

## Patentansprüche

1. Detektionssystem (34) für ferromagnetische Fremdkörper (37) in einem Fluss (42), insbesondere einer Ölfluss in einem Schmierkreislauf (28) einer Turbomaschine (2), wobei das Detektionssystem (34) Folgendes umfasst:
- einen Durchgang (39), der dazu bestimmt ist, vom Fluss (42) durchquert zu werden;
- ein magnetisches Detektionsmodul (38) zum Detektieren von ferromagnetischen Fremdkörpern (37) im Fluss (42), wobei das Modul (38) Folgendes umfasst: eine flussaufwärts gerichtete Hälfte, eine flussabwärts gerichtete Hälfte, einen ersten Magneten (46), eine Spule (44), die dem ersten Magneten (46) zugeordnet und in der flussaufwärts gerichteten Hälfte angeordnet ist;
**dadurch gekennzeichnet, dass** es ferner ein erstes Sieb (60) umfasst, das im Durchgang (39) auf der Seite der flussaufwärts gelegenen Hälfte des Detektionsmoduls (38) zum Filtern von ferromagnetischen Fremdkörpern (37) angeordnet ist.

2. Detektionssystem (34) nach Anspruch 1, **dadurch gekennzeichnet, dass** es ferner einen zweiten Magneten (64) umfasst, der zwischen dem ersten Sieb (60) und dem Detektionsmodul (38) angeordnet ist.

3. Detektionssystem (34) nach einem der Ansprüche 1 bis 2, **dadurch gekennzeichnet, dass** das Detektionsmodul (38) ein Element (48) umfasst, das einen Magnetpol bildet, wobei dieses Element (48) in Kontakt mit dem ersten Magneten (46) ist und die Spule (44) um dieses Element (48) gewickelt ist.

4. Detektionssystem (34) nach Anspruch 3, **dadurch gekennzeichnet, dass** das Element (48) eine Fläche in Kontakt mit dem ersten Magneten (46) und eine Fläche, die dem ersten Sieb (60) und optional dem zweiten Magneten (64) zugewandt ist.

5. Detektionssystem (34) nach einem der Ansprüche 1 bis 4, dadurchgekennzeichnet, dass es ferner ein zweites Sieb (62) umfasst, das im Durchgang (39) zum Abfangen von Schmutz darin angeordnet ist, wobei das Detektionsmodul (38) zwischen dem ersten Sieb (60) und dem zweiten Sieb (62) angeordnet ist.

6. Detektionssystem (34) nach Anspruch 5, **dadurch gekennzeichnet, dass** das erste Sieb (60) erste Netze umfasst und das zweite Sieb (62) zweite Netze umfasst, die feiner sind als die ersten Netze.

7. Detektionssystem (34) nach einem der Ansprüche 1 bis 6, **dadurch gekennzeichnet, dass** es ferner einen dritten Magneten (66) umfasst, der flussabwärts des Detektionsmoduls (38) angeordnet ist.

8. Detektionssystem (34) nach einem der Ansprüche 1 bis 7, **dadurch gekennzeichnet, dass** der Durchgang (39) dazu konfiguriert ist, einen Wirbel im Fluss (42) zu bilden, um ferromagnetische Fremdkörper (37) entsprechend ihrer Masse zu trennen.

9. Detektionssystem (34) nach einem der Ansprüche 1 bis 8 **dadurch gekennzeichnet, dass** es eine Verarbeitungseinheit (36) für das elektrische Signal der Spule (44) umfasst, wobei diese Verarbeitungseinheit (36) dazu ausgelegt ist, die Bewegung eines ferromagnetischen Partikels zu erkennen, wenn das elektrische Signal der Spule (44) eine vordefinierte Schwelle überschreitet.

10. Turbomaschine (2), insbesondere Turbinenstrahltriebwerk, mit einem Schmierkreislauf (28) mit einem Erkennungssystem (34) für ferromagnetische Fremdkörper (37), **dadurch gekennzeichnet, dass** das System (34) einem der Ansprüche 1 bis 9 entspricht, vorzugsweise dem Schmierkreislauf (28) mindestens eine Pumpe (32), einen Tank (30) und eine Flussrichtung umfasst, wobei der erste Bildschirm (60) in einem Abstand und flussaufwärts vom Erkennungsmodul (38) angeordnet ist.

11. Turbomaschine (2) nach Anspruch 10, **dadurch gekennzeichnet, dass** die Pumpe (32) ein Gehäuse umfasst, wobei der Durchgang (39) in dem Gehäuse ausgebildet ist und optional jedes Detektionsmodul (38) von ferromagnetischen Fremdkörpern (37) vom Tank (30) entfernt ist.

12. Verfahren zum Erfassen von ferromagnetischem Fremdkörpern (37) in einem Fluss (42) durch einen Durchgang (39), insbesondere Schmieröl einer Turbomaschine, wobei das Verfahren die folgenden Schritte umfasst: (a) Zirkulation (100) des Flusses beim Durchgangs (39); und (b) Detektion (102) von ferromagnetischen Fremdkörpern im Fluss (42) mit Hilfe eines Detektionssystems (34) für ferromagnetische Fremdkörper (37), **dadurch gekennzeichnet ist, dass** das System einem der Ansprüche 1 bis 9 entspricht, wobei die ferromagnetischen Fremdkörper erste Fremdkörper (50), zweite Fremdkörper (52), dritte Fremdkörper (54) und vierte Fremdkörper (56) möglicherweise in absteigender Reihenfolge enthalten.

13. Verfahren nach Anspruch 12, **dadurch gekennzeichnet, dass** der erste Sieb (60) dazu konfiguriert ist, die ersten Fremdkörper (50) zu blockieren und die zweiten Fremdkörper (52) passieren zu lassen, und dass der erste Magnet (46) dazu konfiguriert ist, die dritten Fremdkörper (54) zu blockieren und optional die zweiten Fremdkörper (52) passieren zu lassen, und dass das System Mittel zum Blockieren der zweiten Fremdkörper (52) beinhaltet.

14. Verfahren nach einem der Ansprüche 12 bis 13 und ein System nach einem der Ansprüche 2 bis 9, **dadurch gekennzeichnet, dass** der zweite Magnet (64) dazu konfiguriert ist, die vierten Fremdkörper (56) zu blockieren und optional die dritten Fremdkörper (54) und die zweiten Fremdkörper (52) passieren zu lassen.

15. Verfahren nach einem der Ansprüche 12 bis 14, **dadurch gekennzeichnet, dass** die Spule (44) des Detektionsmoduls (38) dazu konfiguriert ist, die Bewegung der zweiten Fremdkörper (52) und/oder das Vorhandensein von durch das Detektionsmodul blockiertem dritten Schmutz (54) zu erfassen, wobei optional die zweiten Fremdkörper(52) eine durchschnittliche Größe von 750 µm aufweisen.

## Claims

1. Detection system (34) for ferromagnetic debris (37) in a flow (42), particularly a flow of oil in a lubrication circuit (28) of a turbomachine (2), the detection system (34) comprising:
- a passage (39) intended to be travelled along by the flow (42);
- a magnetic detection module (38) for ferromagnetic debris (37) of the flow (42), the module (38) comprising: an upstream half, a downstream half, a first magnet (46), a coil (44) connected to a first magnet (46) and arranged in the upstream half;
**characterised in that** it furthermore comprises
a first strainer (60) arranged in the passage (39) on the side of the upstream half of the detection module (38) in order to filter ferromagnetic debris (37) from there.

2. Detection system (34) according to claim 1, **characterised in that** it furthermore comprises a second magnet (64) arranged between the first strainer (60) and the detection module (38).

3. Detection system (34) according to one of claims 1 to 2, **characterised in that** the detection module (38) comprises an element (48) forming a magnetic pole, said element (48) being in contact with the first magnet (46) and the coil (44) being wound around said element (48).

4. Detection system (34) according to claim 3, **characterised in that** the element (48) comprises one face in contact with the first magnet (46) and one face opposite the first strainer (60), and may be in contact with the second magnet (64).

5. Detection system (34) according to one of claims 1 to 4, **characterised in that** it furthermore comprises a second strainer (62) arranged in the passage (39) so as to intercept the debris there, the detection module (38) being arranged between the first strainer (60) and the second strainer (62).

6. Detection system (34) according to claim 5, **characterised in that** the first strainer (60) comprises first screens, and the second strainer (62) comprises second screens finer than the first screens.

7. Detection system (34) according to one of claims 1 to 6, **characterised in that** it furthermore comprises a third magnet (66) arranged downstream of the detection module (38).

8. Detection system (34) according to one of claims 1 to 7, **characterised in that** the passage (39) is configured such that it forms a vortex in the flow (42) in such a way as to separate the ferromagnetic debris (37) according to mass.

9. Detection system (34) according to one of claims 1 to 8, **characterised in that** it comprises a processing unit (36) for an electrical signal from the coil (44), said processing unit (36) being adapted to recognise the movement of a ferromagnetic particle when the electrical signal of the coil (44) exceeds a predetermined threshold.

10. Turbomachine (2), particularly a turbojet engine, comprising a lubrication circuit (28) with a detection system (34) for ferromagnetic debris (37), **characterised in that** the system (34) is according to one of claims 1 to 9, preferably the lubrication circuit (28) comprises at least one pump (32), a tank (30) and a direction of flow, the first strainer (60) being arranged at a distance from and upstream of the detection module (38).

11. Turbomachine (2) according to claim 10, **characterised in that** the pump (32) comprises a body, the passage (39) being formed in said body, and each detection module (38) for ferromagnetic debris (37) may be at a distance from the tank (30).

12. Process for detecting ferromagnetic debris (37) in a flow (42) travelling along a passage (39), particularly of the lubricating oil of a turbomachine, the process comprising the following steps: (a) circulation (100) of the flow along the passage (39); and (b) detection (102) of ferromagnetic debris in the flow (42) using a detection system (34) for ferromagnetic debris (37), **characterised in that** this system is according to one of claims 1 to 9; the ferromagnetic debris may comprise, preferably by decreasing order of size, the first debris (50), the second debris (52), the third debris (54) and the fourth debris (56).

13. Process according to claim 12, **characterised in that** the first strainer (60) is configured to block the first debris (50) and to allow the second debris (52) to pass; and **in that** the first magnet (46) is configured to block the third debris (54), and may allow the second debris to pass (52); and **in that** the system comprises means of blocking the second debris (52).

14. Process according to one of claims 12 to 13, and system according to one of claims 2 to 9, **characterised in that** the second magnet (64) is configured to block the fourth debris (56), and may allow the third debris (54) and the second debris (52) to pass.

15. Process according to one of claims 12 to 14, **characterised in that** the coil (44) of the detection module (38) is configured to detect the movement of the second debris (52) and/or the presence of second debris (54) blocked by the detection module, the second debris (52) may have an average size of 750 µm.
